# EUROPEAN PATENT SPECIFICATION

(11) **EP 2 029 594 B1**
(45) Date of publication and mention of the grant of the patent: **20.10.2010**
(21) Application number: 07729313.2
(22) Date of filing: 21.05.2007
(51) Int. Cl.: C07D 471/08, A61K 31/55, A61P 25/00

(54) **NOVEL 1,4-DIAZA-BICYCLO[3.2.2]NONANE DERIVATIVES AND THEIR MEDICAL USE**
NEUARTIGE 1,4-DIAZABICYCLO[3.2.2]NONAN-DERIVATE UND DEREN MEDIZINISCHE VERWENDUNG
NOUVEAUX DÉRIVÉS DE 1,4-DIAZA-BICYCLO[3.2.2]NONANE ET LEUR UTILISATION MÉDICALE

(30) Priority: 23.05.2006 US 802528 P; 23.05.2006 DK 200600704
(43) Date of publication of application: 04.03.2009
(73) Proprietor: NeuroSearch A/S, 2750 Ballerup (DK)
(72) Inventor: PETERS, Dan, DK-2750 Ballerup (DK); OLSEN, Gunnar, M., DK-2750 Ballerup (DK); NIELSEN, Elsebet, Østergaard, DK-2750 Ballerup (DK); TIMMERMANN, Daniel, B., DK-2750 Ballerup (DK); LOECHEL, Steven, Charles, DK-2000 Frederiksberg (DK); CHRISTENSEN, Jeppe, Kejser, DK-2750 Ballerup (DK); DYHRING, Tino, DK-2750 Ballerup (DK)
(86) International application number: PCT/EP2007/054869
(87) International publication number: WO 2007/135120

(56) References cited:
- EP-A1- 1 231 212
- WO-A-00/58311

## Description

### TECHNICAL FIELD

This invention relates to novel 1,4-diaza-bicyclo[3.2.2]nonane derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

EP 1231212 describes pharmaceutical compositions comprising certain 1,4-diazabicyclo[3.2.2]nonane-carboxylic acid derivatives useful as nicotinic alpha-7 receptor agonists, and WO 00/58311 discloses 1,4-diazabicyclo[3.2.2]nonane carboxylic acid derivatives useful as nicotinic alpha-7 receptor agonists.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel 1,4-diaza-bicyclo[3.2.2]-nonane derivatives represented by Formula I 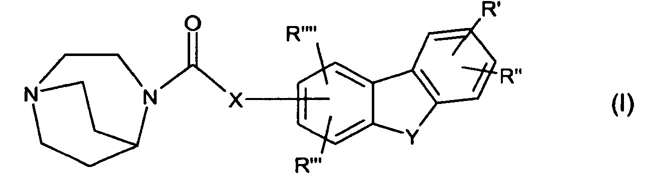 or a pharmaceutically acceptable salt thereof, wherein
X represents O or NH;
Y represents O S, CO, SO₂, CH₂ or CHOH; and
R', R", R"' and R"", independently of each other, represent hydrogen, C₁₋₆-alkyl, C₃₋₇-cycloalkyl, hydroxy, C₁₋₆-alkoxy, halo or trifluoromethyl.

In its second aspect the invention provides pharmaceutical compositions comprising a therapeutically effective amount of the 1,4-diaza-bicyclo[3.2.2]nonane derivatives of the invention, or a pharmaceutically-acceptable addition salt thereof, or a prodrug thereof, together with at least one pharmaceutically-acceptable carrier or diluent.

In a further aspect the invention relates to the use of the 1,4-diazabicyclo[3.2.2]nonane derivatives of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### 1,4-Diaza-bicyclo[3.2.2]nonane Derivatives

In a first aspect novel 1,4-diaza-bicyclo[3.2.2]nonane derivatives are provided. The 1,4-diaza-bicyclo[3.2.2]nonane derivatives of the invention may be represented by Formula I 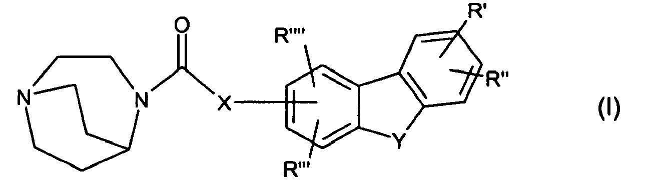 or a pharmaceutically acceptable salt thereof, wherein
X represents O or NH;
Y represents O S, CO, SO₂, CH₂ or CHOH; and
R', R", R"' and R"", independently of each other, represent hydrogen, C₁₋₆-alkyl, C₃₋₇-cycloalkyl, hydroxy, C₁₋₆-alkoxy, halo or trifluoromethyl.

In a first more preferred embodiment the 1,4-diaza-bicyclo[3.2.2]nonane derivative of the invention is a compound of Formula la 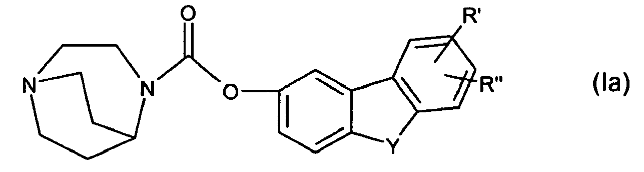 or a pharmaceutically acceptable salt thereof, wherein
X, Y, R' and R" are as defined above.

In a second more preferred embodiment the 1,4-diaza-bicyclo[3.2.2]nonane derivative of the invention is a compound of Formula lb 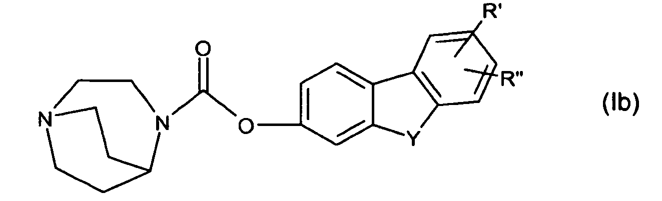 or a pharmaceutically acceptable salt thereof, wherein
X, Y,
R' and R", are as defined above.

In a third more preferred embodiment the 1,4-diaza-bicyclo[3.2.2]nonane derivative of the invention is a compound of Formula I, la or lb, wherein X represents O or NH.

In a more preferred embodiment X represents O.

In another more preferred embodiment X represents NH.

In a fourth more preferred embodiment the 1,4-diaza-bicyclo[3.2.2]nonane derivative of the invention is a compound of Formula I, la or lb, wherein Y represents O, CO, CH₂ or CHOH.

In an even more preferred embodiment Y represents O.

In a still more preferred embodiment Y represents CO.

In a still further more preferred embodiment Y represents CH₂.

In a still further more preferred embodiment Y represents CHOH.

In a fifth more preferred embodiment the 1,4-diaza-bicyclo[3.2.2]nonane derivative of the invention is a compound of Formula I, la or lb, wherein
R' represents hydrogen, alkyl, cycloalkyl, hydroxy, alkoxy, halo or trifluoromethyl; and R", R"' and R"" all represent hydrogen.

In a still further more preferred embodiment R', R", R"' and R"" all represent hydrogen.

In a most preferred embodiment the 1,4-diaza-bicyclo[3.2.2]nonane derivative of the invention is
1,4-Diaza-bicyclo[3.2.2]nonane-4-carboxylic acid 9-oxo-9*H*-fluoren-2-yl ester;
1,4-Diaza-bicyclo[3.2.2]nonane-4-carboxylic acid 9*H*-fluoren-2-yl ester;
1,4-Diaza-bicyclo[3.2.2]nonane-4-carboxylic acid dibenzofuran-3-yl ester;
1,4-Diaza-bicyclo[3.2.2]nonane-4-carboxylic acid (9*H*-fluoren-2-yl)-arnide;
1,4-Diaza-bicyclo[3.2.2]nonane-4-carboxylic acid 9-hydroxy-9*H*-fluoren-2-yl ester;
1,4-Diaza-bicyclo[3.2.2]nonane-4-carboxylic acid dibenzothiophen-4-yl ester;
1,4-Diaza-bicyclo[3.2.2]nonane-4-carboxylic acid 5,5-dioxo-dibenzothiophen-4-yl ester; or
1,4-Diaza-bicyclo[3.2.2]nonane-4-carboxylic acid dibenzofuran-4-yl ester;
or a pharmaceutically acceptable salt thereof.

Any combination of two or more of the embodiments described herein is considered within the scope of the present invention.

### Definition of Substituents

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

In the context of this invention a cycloalkyl group designates a cyclic alkyl group, preferably containing of from three to seven carbon atoms (C₃₋₇-cycloalkyl), including cyclopropyl, cyclobutyl, cyclopentyl, cyclohexyl and cycloheptyl.

In the context of this invention an alkoxy group designates an "alkyl-O-" group, wherein alkyl is defined as a univalent saturated, straight or branched hydrocarbon chain, preferably containing of from one seven carbon atoms (C₁₋₇-alkoxy). Examples of preferred alkoxy groups of the invention include methoxy and ethoxy.

In the context of this invention halo represents fluoro, chloro, bromo or iodo. Thus a trihalomethyl group represents e.g. a trifluoromethyl group, a trichloromethyl group, and similar trihalo-substituted methyl groups.

### Pharmaceutically Acceptable Salts

The 1,4-diaza-bicyclo[3.2.2]nonane derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium). ¹³C, ¹⁴C, ¹³¹I, ¹²⁵I, ¹²³I , and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), and combinations thereof.

### Methods of Producing 1,4-Diaza-bicyclo[3.2.2]nonane Derivatives

The 1,4-diaza-bicyclo[3.2.2]nonane derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the present invention may be useful for the treatment, prevention or alleviation of a cognitive disorder, learning deficit, memory deficits and dysfunction, Down's syndrome, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, fibromyalgia, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

In a more preferred embodiment the compounds of the invention may be useful for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In an even more preferred embodiment the compounds of the invention may be useful for the treatment, prevention or alleviation of diseases, disorders or conditions associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

In a still more preferred embodiment the compounds of the invention may be useful for the treatment, prevention or alleviation of a neurodegenerative disorder, transient anoxia, or induced neuro-degeneration.

In a yet more preferred embodiment the compounds of the invention may be useful for the treatment, prevention or alleviation of an inflammatory disorder, inflammatory skin disorder, acne, rosacea, Crohn's disease, inflammatory bowel disease, ulcerative colitis, or diarrhoea.

In a further preferred embodiment the compounds of the invention may be useful for the treatment, prevention or alleviation of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine containing products such as tobacco, opioids such as heroin, cocaine and morphine, benzodiazepines, benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred compounds of the invention show a biological activity in the submicromolar and micromolar range, i.e. of from below 1 to about 100 µM.

### Pharmaceutical Compositions

In another aspect the invention provides novel pharmaceutical compositions comprising a therapeutically effective amount of the 1,4-diaza-bicyclo[3.2.2]nonane derivatives, derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the 1,4-diaza-bicyclo[3.2.2]nonane derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents. Magnesium sulfate was used as drying agent in the workup-procedures and solvents were evaporated under reduced pressure.

### 1.4-Diazabicyclo[3.2.2]nonane (Intermediate compound)

The title compound was prepared according to J. Med. Chem. 1993 36 2311-2320 (and according to a slightly modified method below).

### 1,4-Diazabicyclo[3.2.2]nonane (Intermediate compound)

To the solution of 1,4-diazabicyclo[3.2.2]nonan-3-one (15.8 g; 113 mmol) in absolute dioxane (130 ml) LiAlH₄ (4.9 g; 130 mmol) was added under argon. The mixture was refluxed for 6 h and then allowed to reach room temperature. To the reaction mixture water (5 ml in 10 ml of dioxane) was added by drops, the mixture was stirred for 0.5 hour and then filtered off via glass filter. The solvent was evaporated and the residue was distilled using Kugelrohr apparatus at 90°C (0.1 mbar) to yield 1,4-diazabicyclo[3.2.2]nonane (11.1 g; 78%) as colourless hygroscopic material.

### 1,4-Diazabicyclo[3.2.2]nonan-3-one (Intermediate compound)

To the solution of 3-quinuclidinone hydrochloride (45 g; 278 mmol) in 90 ml of water hydroxylamine hydrochloride (21 g; 302 mmol) and sodium acetate (CH₃COOHx3H₂O; 83 g; 610 mmol) were added, the mixture was stirred at 70°C for 1 hour and then cooled to 0°C. The separated crystalline material was filtered off (without washing) and dried *in vacuo* to yield 40.0 g of oxime.

The 3-quinuclidinone oxime (40.0 g) was added during 2 hours by small portions to preheated to 120°C polyphosphoric acid (190 g). The temperature of the solution during the reaction was kept at 130°C. After addition of all oxime the solution was stirred for 20 minutes at the same temperature, then transferred to an enamelled vessel and allowed to reach room temperature. The acidic mixture was neutralized by a solution of potassium carbonate (500 g in 300 ml of water), transferred into 2000 ml flask, diluted with 300 ml of water and extracted with chloroform (3 x 600 ml). The combined organic extracts were dried with sodium sulphate, the solvent evaporated and the solid residue dried up *in vacuo* to yield 30.0 g (77%) of the mixture of lactams.

Crystallization of the obtained mixture from 1,4-dioxane (220 ml) gave 15.8 g (40.5%) of 1,4-diazabicyclo[3.2.2]nonan-3-one as colourless large crystals with mp. 211-212°C.

The filtrate was evaporated and the residue was chromatographed on a silica gel (Merck, 9385, 230-400 mesh) column with acetone as eluent. The solvent was evaporated and the residue recrystallized from ethyl etanoate to yield 1,3-diazabicyclo[3.2.2]nonan-4-one (10.2 g; 26%) as colourless fine crystals with mp. 125-126°C.

### Method A

### 1,4-Diaza-bicyclo[3.2.2]nonane-4-carboxylic acid 9-oxo-9H-fluoren-2-yl ester fumaric acid salt (Compound A1)

Phosgen, 20% in toluene (2.87 g, 29.0 mmol) was solved in anhydrous dichloromethane (15 ml) at 0°C. A mixture of 2-hydroxy-fluoren-9-one (1.14 g, 5.81 mmol) and pyridine (0.61 g, 7.73 mmol), solved in dichloromethane (25 ml) was added to the mixture at 0°C. The mixture was stirred for 30 minutes at 0°C and was then allowed to reach room-temperature and was stirred over-night. The reaction-mixture was evaporated and was co-evaporated with toluene (25 ml). The crude intermediate, 1,4-diazabicyclo[3.2.2]nonane (0.73 g 5.81 mmol) and 1,2-dimethoxyethane (40 ml) was stirred at room-temperature for 6 days. Aqueous sodium hydroxide (50 ml, 1 M) was added followed by extraction with chloroform (3 x 30 ml). Chromatography on silica gel with chloroform, 10% methanol and 1% aqueous ammonia as solvent gave the title compound as an oil. The corresponding salt was obtained by addition of a diethyl ether and methanol mixture (9:1) saturated with fumaric acid. Yield 1.40 g, 0.51%. LC-ESI-HRMS of [M+H]+ shows 349.1562 Da. Calc. 349.155218 Da, dev. 2.8 ppm.

### 1,4-Diaza-bicyclo[3.2.2]nonane-4-carboxylic acid 9H-fluoren-2-yl ester fumaric acid salt (Compound A2)

Was prepared from 9*H*-fluoren-3-ol according to Method A. LC-ESI-HRMS of [M+H]+ shows 335.1768 Da. Calc. 335.175953 Da, dev. 2.5 ppm.

### 1,4-Diaza-bicyclo[3.2.2]nonane-4-carboxylic acid dibenzofuran-3-yl ester fumaric acid salt (Compound A3)

Was prepared from dibenzofuran-3-ol according to Method A. LC-ESI-HRMS of [M+H]+ shows 337.1549 Da. Calc. 337.155218 Da, dev. -0.9 ppm.

### 1,4-Diaza-bicyclo[3.2.2]nonane-4-carboxylic acid dibenzothiophen-4-yl ester fumaric acid salt (Compound A4)

Was prepared from dibenzothiophen-4-ol according to Method A. LC-ESI-HRMS of [M+H]+ shows 353,1329 Da. Calc. 353,132374 Da, dev. 1,5 ppm.

### 1,4-Diaza-bicyclo[3.2.2]nonane-4-carboxylic acid 5,5-dioxo-dibenzothiophen-4-yl ester fumaric acid salt (Compound A5)

Was prepared from 5,5-dioxo-dibenzothiophen-4-ol according to Method A. LC-ESI-HRMS of [M+H]+ shows 385,1221 Da. Calc. 385,122204 Da, dev. -0,3 ppm.

### 1,4-Diaza-bicyclo[3.2.2]nonane-4-carboxylic acid dibenzofuran-4-yl ester fumaric acid salt (Compound A6)

Was prepared from dibenzofuran-4-ol according to Method A. LC-ESI-HRMS of [M+H]+ shows 337.1556 Da. Calc. 337.155218 Da, dev. 1.1 ppm.

### Method B

### 1,4-Diaza-bicyclo[3.2.2]nonane-4-carboxylic acid (9H-fluoren-2-yl)-amide fumaric acid salt (Compound B1)

A mixture of 1,4-diazabicyclo[3.2.2]nonane (2.0 g, 15.8 mmol) and methanol (40 ml) was stirred at -50°C. 9-H-fluoren-2-yl-isocyanate (4.3 g, 20.6 mmol) was added at -50°C. The reaction-mixture was allowed to stir at room-temperature for 3 days. Aqueous sodium hydroxide (50 ml, 1 M) was added followed by extraction with chloroform (3 x 50 ml). Chromatography on silica gel with chloroform, 10% methanol and 1% aqueous ammonia as solvent gave the title compound as an oil. The corresponding salt was obtained by addition of a diethyl ether and methanol mixture (9:1) saturated with fumaric acid. Yield 2.04 g, 28%. LC-ESI-HRMS of [M+H]+ shows 334.1919 Da. Calc. 334.191937 Da, dev. -0.1 ppm

### Method C

### 1,4-Diaza-bicyclo[3.2.2]nonane-4-carboxylic acid 9-hvdroxy-9H-fluoren-2-yl ester free base (Compound C1)

A mixture of 1,4-diaza-bicyclo[3.2.2]nonane-4-carboxylic acid 9-oxo-9*H-*fluoren-2-yl ester (0.53 g, 1.51 mmol), sodium borohydride (85 mg, 2.26 mmol) and methanol (20 ml) was allowed to stir for 1 hour at room temperature. The mixture was evaporated. Aqueous sodium hydroxide (20 ml, 1 M) was added followed by extraction by chloroform (3 x 20 ml). Chromatography on silica gel with dichloromethane, 10% methanol and 1% aqueous ammonia as solvent gave the title compound as a solid. Yield 0.53 g (100%). LC-ESI-HRMS of [M+H]+ shows 334.1919 Da. Calc. 334.191937 Da, dev. -0.1 ppm.

### Method D

### Dibenzothiophen-4-ol (Intermediate compound)

A mixture of 4-dibenzothiopheneboronic acid (2.5 g, 11.0 mmol), hydrogen peroxide (2.5 ml, 35%) and ethanol (30 ml, 96%) was stirred at reflux for 2 h. The ethanol was evaporated. Water (50 ml) was added and the mixture was extracted with ethylacetate (2 x 50 ml). Chromatography on silica gel with petroleum and ethyl acetate as solvent gave the title compound. Yield 1.1 g (50%).

### Dibenzofuran-4-ol (Intermediate compound)

Was prepared according to Method D from 4-dibenzofuranboronic acid.

### Method E

### 5,5-Dioxo-dibenzothiophen-4-ol (Intermediate compound)

A mixture of 4-dibenzothiopheneboronic acid (2.6 g, 11.4 mmol), mCPBA (11.2 g, 45.6 mmol), ethanol (10 ml) and chloroform (25 ml) was stirred at 20°C for 15 h. Water (50 ml) was added and the mixture was extracted with ethylacetate (2 x 50 ml). Chromatography on silica gel with dichloromethane, 10% methanol and 1% aqueous ammonia as solvent gave the title compound as a solid. Yield 1.66 g (63%).

## Claims

1. A 1,4-diaza-bicyclo[3.2.2]nonane derivative represented by Formula I 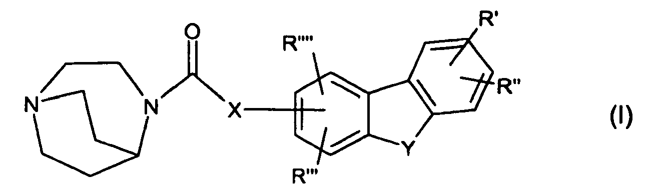 or a pharmaceutically acceptable salt thereof, wherein
X represents O or NH;
Y represents O, S, CO, SO₂, CH₂ or CHOH; and
R', R", R"' and R"", independently of each other, represent hydrogen, C₁₋₆-alkyl, C₃₋₇-cycloalkyl, hydroxy, C₁₋₆-alkoxy, halo or trifluoromethyl.

2. The 1,4-diaza-bicyclo[3.2.2]nonane derivative of claim 1, or a pharmaceutically acceptable salt thereof, wherein X represents O or NH.

3. The 1,4-diaza-bicyclo[3.2.2]nonane derivative of either one of claims 1-2, or a pharmaceutically acceptable salt thereof, wherein Y represents O, CO, CH₂ or CHOH.

4. The 1,4-diaza-bicyclo[3.2.2]nonane derivative of any one of claims 1-3, or a pharmaceutically acceptable salt thereof, wherein
R' represents hydrogen, C₁₋₆-alkyl, C₃₋₇-cycloalkyl, hydroxy, C₁₋₆-alkoxy, halo or trifluoromethyl; and
R", R"' and R"" all represent hydrogen.

5. The 1,4-diaza-bicyclo[3.2.2]nonane derivative of claim 4, or a pharmaceutically acceptable salt thereof, wherein R', R", R"' and R"" all represent hydrogen.

6. The 1,4-diaza-bicyclo[3.2.2]nonane derivative of claim 1, which is
1,4-Diaza-bicyclo[3.2.2]nonane-4-carboxylic acid 9-oxo-9*H*-fluoren-2-yl ester;
1,4-Diaza-bicyclo[3.2.2]nonane-4-carboxylic acid 9*H*-fluoren-2-yl ester;
1,4-Diaza-bicyclo[3.2.2]nonane-4-carboxylic acid dibenzofuran-3-yl ester;
1,4-Diaza-bicyclo[3.2.2]nonane-4-carboxylic acid (9*H*-fluoren-2-yl)-amide; or
1,4-Diaza-bicyclo[3.2.2]nonane-4-carboxylic acid 9-hydroxy-9*H*-fluoren-2-yl ester;
or a pharmaceutically acceptable salt thereof.

7. A pharmaceutical composition comprising a therapeutically effective amount of the 1,4-diaza-bicyclo[3.2.2]nonane derivative of any one of claims 1-6, or a pharmaceutically-acceptable addition salt thereof, or a prodrug thereof, together with at least one pharmaceutically-acceptable carrier or diluent.

8. Use of the 1,4-diaza-bicyclo[3.2.2]nonane derivative of any one of claims 1-6, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

9. The use according to claim 8, wherein the disease, disorder or condition is a cognitive disorder, learning deficit, memory deficits and dysfunction, Down's syndrome, Alzheimer's disease, attention deficit, attention deficit hyperactivity disorder (ADHD), Tourette's syndrome, psychosis, depression, Bipolar Disorder, mania, manic depression, schizophrenia, cognitive or attention deficits related to schizophrenia, obsessive compulsive disorders (OCD), panic disorders, eating disorders such as anorexia nervosa, bulimia and obesity, narcolepsy, nociception, AIDS-dementia, senile dementia, autism, Parkinson's disease, Huntington's disease, Amyotrophic Lateral Sclerosis, anxiety, non-OCD anxiety disorders, convulsive disorders, epilepsy, neurodegenerative disorders, transient anoxia, induced neuro-degeneration, neuropathy, diabetic neuropathy, periferic dyslexia, tardive dyskinesia, hyperkinesia, mild pain, moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury, bulimia, post-traumatic syndrome, social phobia, sleeping disorders, pseudodementia, Ganser's syndrome, pre-menstrual syndrome, late luteal phase syndrome, fibromyalgia, chronic fatigue syndrome, mutism, trichotillomania, jet-lag, arrhythmias, smooth muscle contractions, angina pectoris, premature labour, diarrhoea, asthma, tardive dyskinesia, hyperkinesia, premature ejaculation, erectile difficulty, hypertension, inflammatory disorders, inflammatory skin disorders, acne, rosacea, Crohn's disease, inflammatory bowel disease, ulcerative colitis, diarrhoea, or withdrawal symptoms caused by termination of use of addictive substances, including nicotine containing products such as tobacco, opioids such as heroin, cocaine and morphine, benzodiazepines and benzodiazepine-like drugs, and alcohol.

10. The 1,4-diaza-bicyclo[3.2.2]nonane derivative of any one of claims 1-6, or a pharmaceutically-acceptable addition salt thereof, for use as a pharmaceutical composition/medicament

11. The 1,4-diaza-bicyclo[3.2.2]nonane derivative of any one of claims 1-6, or a pharmaceutically-acceptable addition salt thereof, for use in the treatment, prevention or alleviation of a disease or a disorder or a condition of a mammal, including a human, which disease, disorder or condition is responsive to modulation of cholinergic receptors and/or monoamine receptors.

## Patentansprüche

1. 1,4-Diaza-bicyclo[3.2.2]nonan-Derivat, das durch Formel I wiedergegeben ist 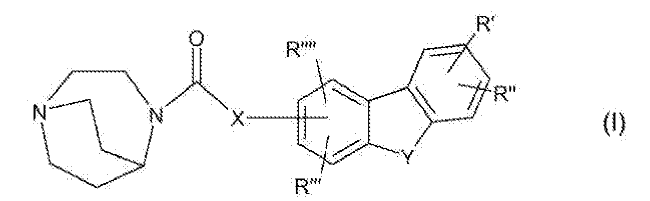 oder ein pharmazeutisch verträgliches Salz davon, worin
X O oder NH bedeutet;
Y O, S, CO, SO₂, CH₂ oder CHOH bedeutet; und
R', R", R"' und R"" unabhängig voneinander Wasserstoff, C₁₋₆-Alkyl, C₃₋₇-Cycoalkyl, Hydroxy, C₁₋₆-Alkoxy, Halogen oder Trifluormethyl bedeuten.

2. 1,4-Diaza-bicyclo[3.2.2]nonan-Derivat nach Anspruch 1, oder ein pharmazeutisch verträgliches Salz davon, worin X O oder NH bedeutet.

3. 1,4-Diaza-bicyclo[3.2.2]nonan-Derivat nach einem der Ansprüche 1-2, oder ein pharmazeutisch verträgliches Salz davon, worin Y O, CO, CH₂ oder CHOH bedeutet.

4. 1,4-Diaza-bicyclo[3.2.2]nonan-Derivat nach einem der Ansprüche 1-3, oder ein pharmazeutisch verträgliches Salz davon, worin
R' Wasserstoff, C₁₋₆-Alkyl, C₃₋₇-Cycloalkyl, Hydroxy, C₁₋₆-Alkoxy, Halogen oder Trifluormethyl bedeutet; und
R", R"' und R"" alle Wasserstoff bedeuten

5. 1,4-Diaza-bicyclo[3.2.2]nonan-Derivat nach Anspruch 4, oder ein pharmazeutisch verträgliches Salz davon, worin R', R", R"' und R"" alle Wasserstoff bedeuten.

6. 1,4-Diaza-bicyclo[3.2.2]nonan-Derivat nach Anspruch 1, welches
1,4-Diaza-bicyclo[3.2.2]nonan-4-carbonsäure-9-oxo-9*H*-fluoren-2-yl-ester;
1,4-Diaza-bicyclo[3.2.2]nonan-4-carbonsäure-9*H*-fluoren-2-yl-ester;
1,4-Diaza-bicyclo[3.2.2]nonan-4-carbonsäure-dibenzofuran-3-yl-ester;
1,4-Diaza-bicyclo[3.2.2]nonan-4-carbonsäure-(9*H*-fluoren-2-yl)-amid; oder
1,4-Diaza-bicyclo[3.2.2]nonan-4-carbonsäure-9-hydroxy-9*H*-fluoren-2-yl-ester ist;
oder ein pharmazeutisch verträgliches Salz davon.

7. Pharmazeutische Zusammensetzung, umfassend eine therapeutisch wirksame Menge des 1,4-Diaza-bicyclo[3.2.2]nonan-Derivats nach einem der Ansprüche 1-6, oder eines pharmazeutisch verträglichen Additionssalzes davon oder eines Prodrugs davon, zusammen mit wenigstens einem pharmazeutisch verträglichen Träger oder Verdünnungsmittel.

8. Verwendung des 1,4-Diaza-bicyclo[3.2.2]nonan-Derivats nach einem der Ansprüche 1-6, oder eines pharmazeutisch verträglichen Additionssalzes davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei diese Krankheit, diese Störung oder dieser Zustand auf die Modulation von cholinergen Rezeptoren und/oder Monoaminrezeptoren anspricht.

9. Verwendung nach Anspruch 8, wobei sich bei der Krankheit, der Störung oder dem Zustand um eine kognitive Störung, ein Lerndefizit, Gedächtnisdefizite und eine Gedächtnisdysfunktion, das Down-Syndrom, die Alzheimer-Krankheit, ein Aufmerksamkeitsdefizit, eine Aufmerksamkeitsdefizit-Hyperaktivitätsstörung (ADHD), das Tourette-Syndrom, eine Psychose, Depression, bipolare Störung, Manie, manische Depression, Schizophrenie, kognitive Defizite oder Aufmerksamkeitsdefizite in Verbindung mit Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, Autismus, die Parkinson-Krankheit, Chorea Huntington, amyotrophe Lateralsklerose, Angst, Nicht-OCD-Angststörungen, konvulsive Störungen, Epilepsie, neurodegenerative Störungen, vorübergehende Anoxie, induzierte Neurodegeneration, Neuropathie, diabetische Neuropathie, periphere Dyslexie, tardive Dyskinesie, Hyperkinesie, milde Schmerzen, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, neuropathische Schmerzen, chronische Kopfschmerzen, zentrale Schmerzen, Schmerzen in Verbindung mit diabetischer Neuropathie, mit posttherapeutischer Neuralgie oder mit peripherer Nervenverletzung, Bulimie, das posttraumatische Syndrom, eine Sozialphobie, Schlafstörungen, Pseudodemenz, das Ganser-Syndrom, das prämenstruelle Syndrom, das Syndrom der späten Lutealphase, Fibromyalgie, das chronische Ermüdungssyndrom, Mutismus, Trichotillomanie, Jetlag, Arrhythmien, Kontraktionen eines glatten Muskels, Angina pectoris, eine Frühgeburt, Diarrhö, Asthma, tardive Dyskinesie, Hyperkinesie, vorzeitige Ejakulation, Erektionsschwierigkeiten, Hochdruck, entzündliche Störungen, entzündliche Hautstörungen, Akne, Rosacea, Morbus Crohn, entzündliche Darmerkrankung, Colitis ulcerosa, Diarrhö oder Entzugssymptome, die durch die Beendigung des Gebrauchs von süchtig machenden Substanzen, einschließlich nikotinhaltiger Produkte wie Tabak, Opioide wie Heroin, Kokain und Morphin, Benzodiazepine und benzodiazepinartiger Drogen bzw. Arzneimittel und Alkohol, verursacht sind, handelt.

10. 1,4-Diaza-bicyclo[3.2.2]nonan-Derivat nach einem der Ansprüche 1-6, oder ein pharmazeutisch verträgliches Additionssalz davon, zur Verwendung als eine pharmazeutische Zusammensetzung/ein Medikament.

11. 1,4-Diaza-bicyclo[3.2.2]nonan-Derivat nach einem der Ansprüche 1-6, oder ein pharmazeutisch verträgliches Additionssalz davon, zur Verwendung bei der Behandlung, Verhütung oder Linderung einer Krankheit oder einer Störung oder eines Zustands eines Säugers, einschließlich eines Menschen, wobei diese Krankheit, diese Störung oder dieser Zustand auf die Modulation von cholinergen Rezeptoren und/oder Monoaminrezeptoren anspricht.

## Revendications

1. Dérivé de 1,4-diaza-bicyclo[3.2.2]nonane représenté par la formule I 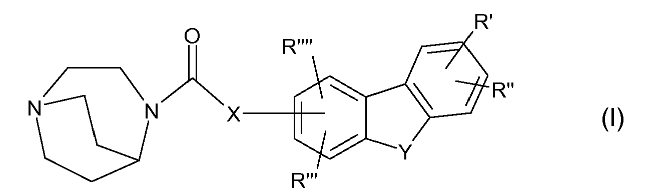 ou un sel pharmaceutiquement acceptable de celui-ci, dans lequel
X représente O ou NH ;
Y représente O, S, CO, SO₂ CH₂ ou CHOH ; et
R', R", R"' et R"", indépendamment les uns des autres, représentent un hydrogène, un alkyle en C₁₋₆, un cycloalkyle en C₃₋₇, un hydroxy, un alcoxy en C₁₋₆, un halogéno ou un trifluorométhyle.

2. Dérivé de 1,4-diaza-bicyclo[3.2.2]nonane selon la revendication 1, ou un sel pharmaceutiquement acceptable de celui-ci, dans lequel X représente O ou NH.

3. Dérivé de 1,4-diaza-bicyclo[3.2.2]nonane selon l'une quelconque des revendications 1-2, ou un sel pharmaceutiquement acceptable de celui-ci, dans lequel Y représente O, CO, CH₂ ou CHOH.

4. Dérivé de 1,4-diaza-bicyclo[3.2.2]nonane selon l'une quelconque des revendications 1-3, ou un sel pharmaceutiquement acceptable de celui-ci, dans lequel
R' représente un hydrogène, un alkyle en C₁₋₆, un cycloalkyle en C₃₋₇, un hydroxy, un alcoxy en C₁₋₆, un halogéno ou un trifluorométhyle ; et
R", R"' et R"" représentent tous un hydrogène.

5. Dérivé de 1,4-diaza-bicyclo[3.2.2]nonane selon la revendication 4, ou un sel pharmaceutiquement acceptable de celui-ci, dans lequel R', R", R"' et R"" représentent tous un hydrogène.

6. Dérivé de 1,4-diaza-bicyclo[3.2.2]nonane selon la revendication 1, qui est
l'ester 9-oxo-9*H*-fluorén-2-ylique de l'acide 1,4-diaza-bicyclo[3.2.2]-nonane-4-carboxylique ;
l'ester 9*H*-fluorén-2-ylique de l'acide 1,4-diaza-bicyclo[3.2.2]nonane-4-carboxylique ;
l'ester dibenzofuran-3-ylique de l'acide 1,4-diaza-bicyclo[3.2.2]-nonane-4-carboxylique ;
l'amide (9*H*-fluorén-2-ylique) de l'acide 1,4-diaza-bicyclo[3.2.2]-nonane-4-carboxylique ; ou
l'ester 9-hydroxy-9*H*-fluorén-2-ylique de l'acide 1,4-diaza-bicyclo-[3.2.2]nonane-4-carboxylique ;
ou un sel pharmaceutiquement acceptable de celui-ci.

7. Composition pharmaceutique comprenant une quantité thérapeutiquement efficace du dérivé de 1,4-diaza-bicyclo[3.2.2]nonane selon l'une quelconque des revendications 1-6, ou d'un sel d'addition pharmaceutiquement acceptable de celui-ci, ou d'un prodrogue de celui-ci, conjointement avec au moins un véhicule ou diluant pharmaceutiquement acceptable.

8. Utilisation du dérivé de 1,4-diaza-bicyclo[3.2.2]nonane selon l'une quelconque des revendications 1-6, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'une affection d'un mammifère, incluant un être humain, laquelle maladie, lequel trouble ou laquelle affection réagit à une modulation des récepteurs cholinergiques et/ou des récepteurs des monoamines.

9. Utilisation selon la revendication 8, dans laquelle la maladie, le trouble ou l'affection est un trouble cognitif, un défaut d'apprentissage, des déficiences et un dysfonctionnement de la mémoire, un syndrome de Down, une maladie d'Alzheimer, une déficience de l'attention, un trouble d'hyperactivité avec déficience de l'attention (THADA), un syndrome de Gilles de la Tourette, une psychose, une dépression, un trouble bipolaire, une manie, une manie-dépression, une schizophrénie, des déficiences cognitives ou de l'attention liées à une schizophrénie, des troubles obsessionnels compulsifs (TOC), des troubles paniques, des troubles de l'alimentation tels qu'une anorexie mentale, une boulimie et une obésité, une narcolepsie, une nociception, une démence associée au SIDA, une démence sénile, un autisme, une maladie de Parkinson, une chorée de Huntington, une sclérose latérale amyotrophique, une anxiété, des troubles d'anxiété non TOC, des troubles convulsifs, une épilepsie, des troubles neurodégénératifs, une anoxie transitoire, une neuro-dégénérescence induite, une neuropathie, une neuropathie diabétique, une dyslexie périphérique, une dyskinésie tardive, une hyperkinésie, une douleur légère, une douleur modérée ou sévère, une douleur de caractère aigu, chronique ou récurrent, une douleur provoquée par une migraine, une douleur postopératoire, une douleur de membre fantôme, une douleur inflammatoire, une douleur neuropathique, une céphalée chronique, une douleur centrale, une douleur liée à une neuropathie diabétique, à une névralgie post-thérapeutique ou à une blessure de nerf périphérique, une boulimie, un syndrome post-traumatique, une phobie sociale, des troubles du sommeil, une pseudo-démence, un syndrome de Ganser, un syndrome prémenstruel, un syndrome de la phase lutéale tardive, une fibromyalgie, un syndrome de fatigue chronique, un mutisme, une trichotillomanie, un décalage horaire, des arythmies, des contractions de muscle lisse, une angine de poitrine, un travail prématuré, une diarrhée, un asthme, une dyskinésie tardive, une hyperkinésie, une éjaculation prématurée, une difficulté d'érection, une hypertension, des troubles inflammatoires, des troubles cutanés inflammatoires, une acné, une rosacée, une maladie de Crohn, une maladie intestinale inflammatoire, une rectocolite ulcérative, une diarrhée, ou des symptômes de sevrage provoqués par l'arrêt d'utilisation de substances toxicomanogènes incluant les produits contenant de la nicotine comme le tabac, les opioïdes tels que l'héroïne, la cocaïne et la morphine, les benzodiazépines et les substances médicamenteuses de type benzodiazépine, et l'alcool.

10. Dérivé de 1,4-diaza-bicyclo[3.2.2]nonane selon l'une quelconque des revendications 1-6, ou un sel d'addition pharmaceutiquement acceptable de celui-ci, pour l'utilisation en tant que composition pharmaceutique/médicament.

11. Dérivé de 1,4-diaza-bicyclo[3.2.2]nonane selon l'une quelconque des revendications 1-6, ou un sel d'addition pharmaceutiquement acceptable de celui-ci, pour l'utilisation dans le traitement, la prévention ou l'atténuation d'une maladie ou d'un trouble ou d'une affection d'un mammifère, incluant un être humain, laquelle maladie, lequel trouble ou laquelle affection réagit à une modulation des récepteurs cholinergiques et/ou des récepteurs des monoamines.
